(19) Europäisches Patentamt
European Patent Office
Office européen des brevets

(11) **EP 4 232 877 B1**

(12) **EUROPEAN PATENT SPECIFICATION**

(45) Date of publication and mention
of the grant of the patent:
**18.12.2024 Bulletin 2024/51**

(21) Application number: **21791349.0**

(22) Date of filing: **14.10.2021**

(51) International Patent Classification (IPC):
**G05B 23/02** (2006.01)          **G16H 40/40** (2018.01)

(52) Cooperative Patent Classification (CPC):
**G05B 23/0283; G16H 40/40;** G06N 3/045;
G06N 20/10

(86) International application number:
**PCT/EP2021/078380**

(87) International publication number:
**WO 2022/084127 (28.04.2022 Gazette 2022/17)**

(54) **MULTI-CRITERIA FAIR QUEUEING OF ALERTS**

FAIRE MULTIKRITERIENWARTESCHLANGEN VON ALARMEN

MISE EN FILE D'ATTENTE ÉQUITABLE À MULTI-CRITÈRES D'ALERTES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.10.2020 US 202063094955 P**

(43) Date of publication of application:
**30.08.2023 Bulletin 2023/35**

(73) Proprietor: **Koninklijke Philips N.V.
5656 AG Eindhoven (NL)**

(72) Inventors:
• **KORST, Johannes Henricus Maria
5656 AE Eindhoven (NL)**

• **BARBIERI, Mauro
5656 AE Eindhoven (NL)**
• **PRONK, Serverius Petrus Paulus
5656 AE Eindhoven (NL)**
• **PETERS, Marc André
5656 AE Eindhoven (NL)**
• **GAO, Qi
5656 AE Eindhoven (NL)**

(74) Representative: **Philips Intellectual Property &
Standards
High Tech Campus 52
5656 AG Eindhoven (NL)**

(56) References cited:
**EP-A1- 3 379 356          US-A1- 2011 121 969
US-A1- 2020 185 085**

**Description**

<u>FIELD</u>

The following relates generally to the maintenance arts, medical device arts, medical device maintenance arts, weighted fair queueing arts, multi-criteria optimization arts, and related arts.

<u>BACKGROUND</u>

The maintenance of medical imaging systems (and other medical devices such as patient monitoring systems) consists of multiple types of maintenance activities. The maintenance can contain planned activities where a field service engineer (FSE) visits the hospital to oil, clean, calibrate, etc. the system at regular intervals (e.g. once or twice every year, or with a frequency that is determined by the usage of the system, or dynamically scheduled based on remotely monitoring the condition of the system). In addition, there are corrective maintenance activities that are initiated as a reaction on an issue reported by the hospital. If the issue is severe, then this may result in unplanned down time of the system. The system may not be operational until the issue is fixed again, either remotely or by way of an on-site visit by an FSE. Unplanned down time can lead to considerable costs for the hospital as no examinations can be scheduled for some time. It can also lead to patient dissatisfaction, as examinations may have to be rescheduled to a later time.

In recent years, in addition to the above-mentioned maintenance activities, predictive maintenance activities are used more often, in an effort to avoid unplanned downtime. For various parts of a medical imaging system, predictive models have been made that aim to predict when a part is likely to fail in the near future, so that the part can be replaced preventively before it actually fails. These predictive models may be constructed by using a machine learning (ML) algorithm that, on the basis of a training set of historical cases, builds a predictive model. For a given subsystem or part $p$, such a predictive model will estimate, for a given system $s$ and a given time window $[t, t + w]$, the probability $Pr(p, s, t)$ that subsystem or part $p$ of system $s$ will fail in this time window. These estimated probabilities can next be used to determine whether it makes sense to preventively replace subsystem/part $p$ in the coming week or weeks. The predictive models use log event data that the system produces to be analyzed remotely. Log event data may contain sensor measurements as well as log events in the form of low-level error and warning messages.

Once a predictive model has been tested to perform at a sufficient performance level (taking into account the probability and cost of false positives as well as false negatives), it can be deployed to monitor many systems in the field. The model can be run on recent log event data of each of the systems at regular intervals, e.g. once every hour or day, or it can be triggered dynamically by the availability of new and relevant data. If for a system s, it concludes that $Pr(p, s, t)$ exceeds a certain threshold, it can raise an alert. Alternative strategies, such as exceeding a threshold at least $k$ times in $l$ successive time units can also be used to raise an alert.

Specialized remote monitoring engineers (RMEs) are trained to review the raised alerts via a computer program that shows a ranked list of recent alerts. To each alert of type $a$ a priority $P(a)$ is associated, so that all alerts that are raised by the multiple predictive models are ranked by priority, e.g. the list of recent alerts is ranked by non-increasing priority: at the top of the list the alert(s) with the highest priority are shown and the priority decreases when going down the list. The RMEs typically consider the alerts in a top down fashion. The type $a$ of an alert is based on the predictive model, and likewise the priority $P(a)$ is based on the parameters of the predictive model, such as accuracy, false positive rate (FPR), and window size w.

A disadvantage of only using the priority of alerts to rank them in the alert list is that it does not take into account the many other aspects that could play a role in sensibly ordering the alerts. For example, in addition to a priority, it might be useful to take into account the remaining time in which the service organization has to react, in order not to risk that the replacement of the part is still scheduled too late to avoid that it actually fails. Hence, in addition to a priority, there is also a deadline attached. Furthermore, there can be other aspect that are not taken into account by an alerting system that employs priority ranking, such as the type of service contract the hospital has for the given system.

To emphasize the deadlines, alerts can be ranked on the list using for example an earliest-deadline-first algorithm. However, this approach only considers the deadline and not the priority. In addition, there are various scheduling algorithms for which the priority is directly based on the deadline, such that high priority activities by definition have a short deadline.

Another disadvantage of an alerting system that ranks alerts by priority is that some alerts may never be observed by the RMEs, as they may be ranked so low that they never arrive at the top of the list. It can be that some predictive models do no longer work in the circumstances for which they have originally been designed. For example, the data that is used may have changed after a new software release. As a result, such a model may start generating more alerts. However, if the predictive model has a low priority, then this may be only detected in a late stage. Each time a RME evaluates the correctness of an alert generated by a predictive model, the predictive model is evaluated whether or not it still works as anticipated during its construction. Hence, it would be advantageous for the alerts generated by all models to be evaluated regularly, be it at different rates.

Yet another disadvantage is that a model priority-based ordering does not take into account that some hospitals may have multiple similar imaging systems, such that they can reschedule examinations to another similar system, while other hospitals may have only one system of the given type of system, so that due to a failure of this single system they cannot perform these examinations as long as the issue is not resolved. Hence a high ranking for an alert relating to an imaging system when the hospital has several other imaging systems of that modality may not be optimal. Similarly, a low ranking for an alert relating to an imaging system, which is the only one of that modality possessed by the hospital, may also be sub-optimal.

The following discloses certain improvements to overcome these problems and others.

A document EP 3379356 A1 discloses a method for a lithographic apparatus. The method determines a causal relationship between events (A-D) in a plurality of parameter time series associated with an industrial process, identifying at least a first event (A, B, D) associated with a parameter excursion event; identifying at least a second event (C) associated with a failure event; and establishing the causal relationship between the first event (A, B, D) and the second event (C) by using a determined value for the transfer entropy between the first event (A, B, D) and second event (C).

## SUMMARY

The invention is set out in the appended set of claims.

One advantage resides in reducing downtime of a medical imaging device.

Another advantage resides in ranking medical imaging device alerts with criteria other than priority.

Another advantage resides in ranking medical imaging device alerts for multiple modalities.

Another advantage resides in ranking medical device alerts with multiple criteria.

Another advantage resides in a remote expert reviewing a ranked list of alerts and providing assistance to a local operator of a medical device.

A given embodiment may provide none, one, two, more, or all of the foregoing advantages, and/or may provide other advantages as will become apparent to one of ordinary skill in the art upon reading and understanding the present disclosure.

## BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

FIGURE 1 diagrammatically illustrates an illustrative system for generating a ranked list of alerts from logs files of medical electronic devices in accordance with the present disclosure.
FIGURE 2 shows exemplary flow chart operations of the system of FIGURE 1.

## DETAILED DESCRIPTION

A medical imaging device service department has a staff of RMEs who review automatically generated alerts issued based on the machine logs of imaging devices assigned to them. To this end, predictive models are applied to the log data. For a given subsystem/part $p$, a predictive model outputs a probability $Pr(p, s, t)$ that the subsystem/part $p$ will fail in a time window $[t, t + w]$ where $w$ is the window width. In a typical scenario, an alert is generated if this probability is higher than some threshold (which may be a different threshold for different models). Currently, each alert ($a$) is assigned a priority $P(a)$, and the RME is presented with a list of alerts for the assigned machines ranked by the probabilities $P(a)$.

A problem with this approach arises in that the probabilities do not take into account some other relevant factors (e.g. deadline for addressing the alert, effect on the hospital or radiology lab if the part actually fails, et cetera). Further, the priorities are usually fixed and so the top-ranked alerts will remain top-ranked day-by-day. As a given RME may receive dozens of alerts of varying priority each day, this can result in the RME missing alerts of lower priority that never show up near the top of the list.

To address these problems, the following discloses adapting a class of wired or wireless communication queuing methods known as fair queuing methods to the alert ranking problem. Fair queuing methods allocate successive communication channel slots to data streams in a manner that provides each data stream with predetermined share of the channel due to that data stream, but does so in a way that distributes the allocations so that the highest priority data streams do not receive all the channel slots. To do so, the following describes a credit-based fair queuing method in which each data stream has a maintained amount of credit (suitably initially set to their respective shares). For each iteration (corresponding to a slot), a credit for each channel is increased by its share. The channel with the highest accumulated credit is assigned the slot and its credit is decreased by 1.00. As the shares of all data streams sharing the channel are normalized to sum up to 1.00, this means that the total credit over all channels is unchanged for each iteration (since the added credit in each iteration sums to 1.00 and exactly this amount 1.00 is subtracted from the channel receiving the slot).

In adapting the fair queuing for ranking alerts, each alert corresponds to a data stream, the channel corresponds to the ranked list, and each channel slot corresponds to a position in the ranked list. In one approach, the (normalized) priority of each alert corresponds to the share for that alert, and the fair queuing thus ensures that the highest priority alert is not repeatedly ranked at the top of the ranked alerts list.

However, this approach does not account for the various other factors that might be usefully considered in the ranking.

To account for multiple factors in the ranking, in some embodiments disclosed herein, an improved fair queuing method referred to as multi-criterion fair queuing is implemented. In this approach, each criterion is assigned a set of assumable discrete criterion share values that sum to 1.00 (that is, the assumable criterion share values for each criterion are normalized). The criterion share values are denoted as $v_{ij}$ where $i$ indexes the criteria and $j$ indexes the assumable values for the criterion indexed by $i$. Then, alert categories $c(v_{1,[]}, v_{2,[]}, ...)$ are defined as all possible combinations of criterion share values $(v_{1,[]}, v_{2,[]}, ...)$ for all criteria. As an example, if there are two criteria, one having two possible criterion share values $v_{1,1}$ and $v_{1,2}$ and the other having three possible criterion share values $v_{2,1}$, $v_{2,2}$, and $v_{2,3}$, then there are 2x3=6 alert categories. The share for each alert category is computed as a product of criterion share values for that alert category. Due to the normalization of the assumable criterion share values of each criterion, this results in the computed shares for the alert categories summing to 1. Hence, the resulting computed shares can be used in the fair queuing algorithm as normal. (Note, if a particular criterion share value has no occurrences in the list of alerts, then that criterion share value is set to 0.0 and a renormalization is performed).

Advantageously, the criterion share values for a given criterion can be redistributed or completely redesigned without impacting any of the other criteria, so long as the updated criterion share values are re-normalized. Even more, if the value of any criterion cannot be determined, then it can be effectively removed by setting the criterion share value to 1, as this has no impact on the product of the (remaining) criterion share values.

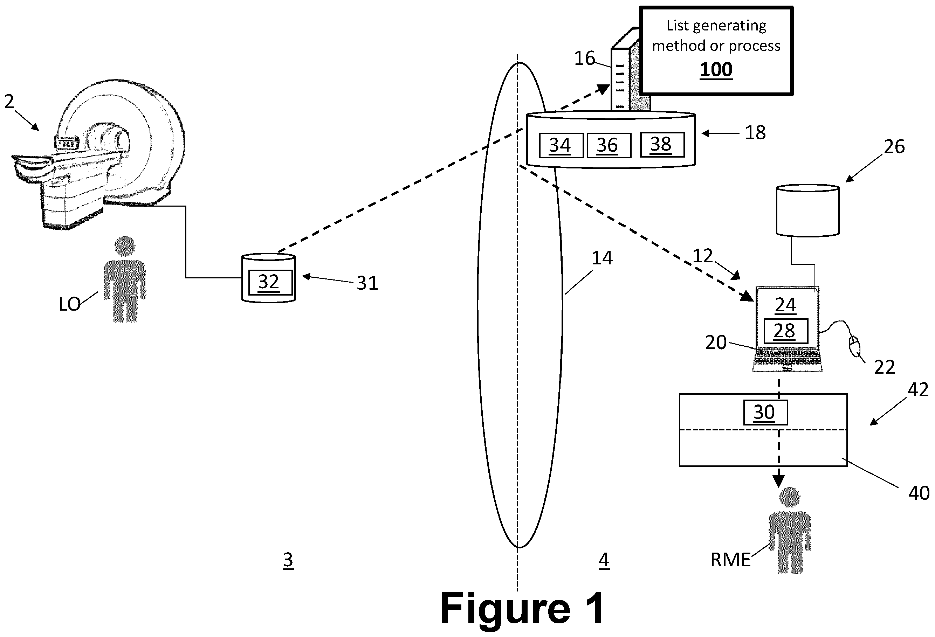

With reference to FIGURE 1, an apparatus or system 1 for generating a ranked list of alerts from logs files of medical electronic devices 2 (e.g., medical imaging devices) is shown. As shown in FIGURE 1, a local operator **LO,** who operates a medical imaging device (also referred to as an image acquisition device, imaging device, and so forth) **2,** is located in a medical imaging device bay **3,** and the remote monitoring engineer **RME** is disposed in a remote service location or center **4.** The remote location **4** is typically a site owned or leased or otherwise controlled by the imaging device vendor or other imaging device service provider.

A single image acquisition device **2** is shown by way of illustration, which can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device **2** may also be a hybrid imaging device such as a PET/CT or SPECT/CT imaging system. While a single image acquisition device **2** is shown by way of illustration in FIGURE 1, more typically the service location or center **4** will provide monitoring and maintenance servicing for a fleet of imaging devices, e.g. for all or a subset of medical imaging devices sold by the vendor under service contracts between the customers (e.g., owner or operator of the imaging bays **3**). The fleet that is serviced by the service location or center **4** may number in the dozens or hundreds or more imaging devices. A given customer, e.g. a medical imaging laboratory, will have one or (more typically) multiple image acquisition devices, which may be of the same and/or different imaging modalities. For example, if a hospital performs many CT imaging examinations and relatively fewer MRI examinations and still fewer PET examinations, then the hospital's imaging laboratory (sometimes called the "radiology lab" or some other similar nomenclature) may have three CT scanners, two MRI scanners, and only a single PET scanner. This is merely an example. Moreover, the remote service center **4** will typically provide service to multiple hospitals or other customers.

In a typical scenario, the various imaging devices **2** that are serviced by the service center **4** automatically generate machine logs that record operational information pertaining to the imaging devices **2,** such as scans performed, hardware and parameters used in the scans, device configuration changes, and so forth. By way of one non-limiting illustrative example, in the case of a CT scanner, the machine log may store X-ray tube usage including time intervals the X-ray tube is energized and the parameters such as X-ray tube voltage and current. These logs are automatically transferred to the service center **4** by way of a communication link **14,** such as the Internet possibly augmented by local area networks at the customer end **3** and/or at the service center end **4.** These logs may for example be transferred on a daily basis or more or less frequently and serve as input data for predictive models run at the service center **4** to generate alerts.

FIGURE 1 also shows, in the remote service center **4** including one or (typically) more workstations **12,** such as an electronic processing device, a smartphone, a tablet, a workstation computer, or more generally computers manned by **RME**s, which are operatively connected to receive data related to alerts for the medical imaging device **2,** as described in more detail below. Additionally or alternatively, the remote workstation **12** can be embodied as a server computer or a plurality of server computers **16,** e.g. interconnected to form a server cluster, cloud computing resource, or so forth. The workstation **12** includes typical components, such as an electronic processor **20** (e.g., a microprocessor), at least one user input device (e.g., a mouse, a keyboard, a trackball, and/or the like) **22,** and at least one display device **24** (e.g. an LCD display, plasma display, cathode ray tube display, and/or so forth). In some embodiments, the display device **24** can be a separate component from the workstation **12.** The display device **24** may also comprise two or more display devices.

The electronic processor **20** is operatively connected with a one or more non-transitory storage media **26.** The non-transitory storage media **26** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the workstation **12,** various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a graphical user interface (GUI) **28** for display on the remote expert display device **24.**

The service center **4** may be housed in a single physical location, or may be geographically distributed. The service center **4** includes a server computer **16** (or a cluster of servers, cloud computing resource comprising servers, or so forth) which is programmed to perform functions such as applying predictive models to machine log data generated by the imaging devices **2** serviced by the center **4** to generate alerts, and provide user interfaces (UIs) on the workstations **12** which are staffed by RMEs. The server computer **16** is operatively connected with a one or more non-transitory storage media **18.** The non-transitory storage media **18** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the server computer **16,** various combinations thereof, or so forth. It is to be understood that any reference to a non-transitory medium or media **18** herein is to be broadly construed as encompassing a single medium or multiple media of the same or different types. Likewise, the server computer **16** may be embodied as a single electronic processor or as two or more electronic processors. The non-transitory storage media **18** stores instructions executable by the server computer **16.**

Furthermore, as disclosed herein the server computer **16** performs a method or process **100** for generating a ranked list of alerts **30** from files **32** of the illustrative medical imaging device **2** stored in a non-transitory computer readable medium **31** of the medical device controller **10.** The files **32** can include, for example, machine logs files, base data (which specifies how many medical devices of a given type a given customer has), contract data (i.e., between the customer and the servicing vendor), or any other suitable type of data. In addition, the files can include data related to the capabilities of the respective remote monitoring engineer **RMEs.** The server computer **16** is configured to retrieve the files **32** from the medical device controller **10** via the communication link **14,** e.g. on a daily basis or more or less frequently. In this manner, the remote monitoring engineer **RME** can review the ranked list of alerts **30,** and can take appropriate action, such as attempting to remediate the alert remotely or contacting the customer to schedule a visit by a field service engineer (FSE) to address the alert on-site.

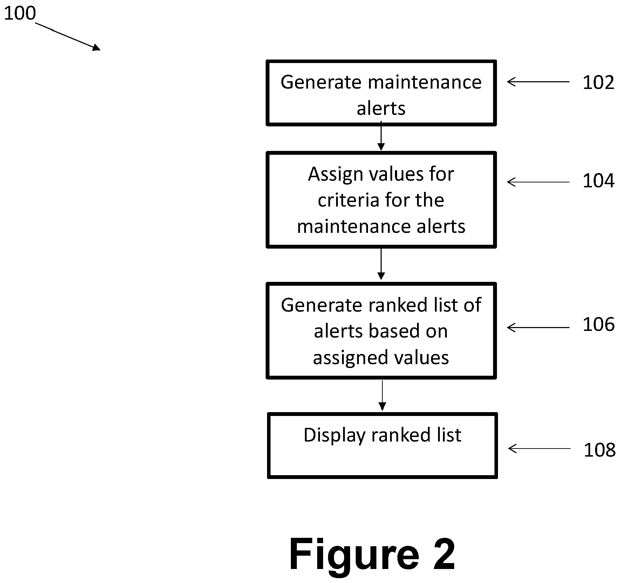

With reference to FIGURE 2, and with continuing reference to FIGURE 1, the server computer **16** is configured as described above to perform the method **100** for generating the ranked list of alerts **30** from the files **32** of the medical imaging device **2.** The non-transitory storage medium **18** stores instructions which are readable and executable by the server computer **16** to perform disclosed operations including performing the method or process **100.** In some examples, the method **100** may be performed at least in part by cloud processing.

At an operation **102,** one or more predictive models **34** are applied to data of the retrieved files **32** to generate the maintenance alerts **30** for the medical electronic devices **2.** The predictive models **34** are stored in the non-transitory computer readable medium **18** of the server computer **16.** The predictive models **34** may include one or more statistical models, e.g. statistically predicting remaining X-ray tube life based on the time-integrated tube current, time-integrated tube voltage, and/or other measurable parameters of the X-ray tube using historical data for past X-ray tube installs and failures. The predictive models **34** may include one or more artificial intelligence (AI) models, e.g. X-ray detector array (or array sub-module) replacement time may be predicted by applying a convolutional neural network (CNN) to images (if available to the server **16** in the data **32** or from another source) to detect image artifacts indicative of impending performance degradation that the CNN has been trained on historical data to detect. Other types of AI, such as support vector machines (SVMs) may be trained to generate alerts based on training on historical data. The predictive models **34** may include less sophisticated models, such as for example an alert being based solely on time-since-install, based on usable part life information provided by the part manufacturer. In this case, the predictive model reads the install date of the part from the machine log and determines the alert based on the time elapsed since that install date. These are merely some non-limiting illustrative examples.

In operations **104, 106,** a credit-based queuing process **38** is applied to rank the alerts. At an operation **104,** discrete values are assigned for a set of criteria **36** (stored in the non-transitory computer readable medium **18** of the server computer **16)** to each maintenance alert **30.** The set of criteria **36** can include for example, a predictive model criterion whose assigned value for a maintenance alert **30** corresponds to the predictive model **34** that generated the maintenance alert, and a timeframe assigned to the maintenance alert by the predictive model **34.** In some embodiments, the set of criteria **36** can also include a criterion based on a maintenance contract for the electronic device **2** for which the maintenance alert **30** is generated, such as for example, the terms of the maintenance contact, a nature of the contract which the electronic device **2,** is under, and so forth. In another example, the set of criteria **36** can include a criterion based on a satisfaction level of an owner of the electronic device **2** for which the maintenance alert **30** is generated. For example, the satisfaction level can include a criterion based on potential downtime of the electronic device **2.** To do so, the credit-based queuing process **38** can analyze the generated maintenance alert **30** with machine learning algorithms to predict possible downtime. A higher downtime can result from a complexity of the service case (i.e., more than one probable root cause or more than one set of contributing parts); available FSE resources in the area; availability of the part (in general or in the region), and so forth. In other embodiments, when the medical imaging devices **2** comprise different modalities (e.g., MR and CT), the set of criteria **36** can also include a criterion based on the imaging modality of the medical imaging device **2** (e.g., type of modality, a number of medical imaging devices, and so forth) for which the maintenance alert **30** is generated. In another example, the set of criteria **36** can include a criterion based on a volume of examinations using the medical imaging device (s) **2** relative to the number of medical imaging devices. This volume of examinations can be analyzed retrospectively and/or prospectively.

The maintenance alert **30** has a share computed as a product of the assigned discrete values. To do so, each criterion of the plurality of criteria **36** assume discrete values of a set of discrete values in which the sum of the discrete values of the set of discrete values equals one (e.g., 1.00). That is, each criterion can have an initial share value of less than one. In some examples, the set of discrete values can be recomputed for a single criterion while ensuring the sum of the discrete values of the set of discrete values remains equal to one. Maintenance alerts **30** having a value of zero, or an interminable value, can be discarded.

With further reference to FIGURE 2, at an operation **106,** the credit-based queuing process **38** is performed to assign the maintenance alerts **30** to slots **40** of a ranked list **42.** In the credit-based queuing process **38,** the maintenance alerts **30** accumulate credits in accord with the shares computed for the respective maintenance alerts. In some embodiments, the credit-based queuing process **38** comprises a credit-based fair queuing process. In each iteration of the credit-based fair queuing process **38,** the credit for each maintenance alert **30** is increased by an amount equal to the share computed for the maintenance alert. The maintenance alert **30** having the highest accumulated credit is assigned to a current slot **40** of the ranked list **42.** Once this maintenance alert **30** is assigned to the slot **40,** the credit of the assigned maintenance alert is decreased by a predetermined amount, such as 1.00. The credit-based fair queuing process **38** is repeated until all maintenance alerts **30** are assigned to corresponding slots **40** in the ranked list **42.**

Referring back to FIGURES 1 and 2, at an operation **108,** the ranked list **42** is displayed on the display device **24** of the remote workstation **12** in accordance with the assignments of the maintenance alerts to the slots of the ranked list. The remote expert **RME** can review the list **42,** and provide assistance or bring attention to the local operator **LO** of the respective medical imaging devices **2** to which the maintenance alerts **30** on the list **42** correspond to.

EXAMPLE

The following describes in more detail the credit-based queuing process **38** performed by the method **100.** The credit-based fair-queueing process **38** is generalized to a multi-criteria variant. A credit-based fair queuing process **38** can be used if multiple processes have to share a scarce resource in a fair way (e.g., the attention of the remote monitoring engineer **RME**). It is often used in the context of sharing a single communication channel *c* by *n* data streams, numbered 1, 2, ..., *n.* The data stream *i* gets a fraction $s_i$ of the total capacity of channel *c,* with $\sum_{i=1}^{n} s_i = 1$.

The communication channel consists of successive slots **40,** numbered 1,2, ..., and so forth, where each slot is assigned to one of the *n* data streams in the following way. During the assignment of slots **42** to the data streams, a credit $c_i$ is maintained for each data stream *i.* $c_i[t]$ denotes the credit of data stream *i* at the start of slot *t.* All data streams start with a zero credit, i.e., $c_i[1] = 0$ for all *i.* At the start of each slot **40,** the credit of each data stream *i* is increased by $s_i$, the share that data stream *i* is entitled to. Next, the slot **40** is assigned to the data stream that has the highest credit and the credit of this data stream is lowered by 1. Hence, during each slot **40** the sum of all credits is increased by $s_1 +$

$s_2 + \cdots + s_n = 1$, and decreased again by 1, by decreasing the credit of the data stream that received the current slot by 1. Hence, as $\sum_{i=1}^{n} c_i[1] = 0$, then $\sum_{i=1}^{n} c_i[j] = 0$ for all $j$ = 1,2, .... As a consequence, each data stream on average receives its fair share of the slots **40,** and the credit for each data stream at the start of each slot remains bounded. As a consequence, also data streams that only have a small share, e.g., a share $s_i \ll \dfrac{1}{n}$ still gets a fraction of the slots, on average, that equals this share. The fair queueing algorithm **38** is slightly more complicated than this if one of the data streams does not have any data packets to transmit.

As disclosed herein, this principle can be used to rank the alerts **30** of multiple predictive models **34.** This can be performed for a single criterion (as an illustrative example) as follows. There are $n$ predictive models **34,** numbered 1, 2, ..., $n$, where the priority of alerts **30** from model $i$ is given by $p_i$, with $p_i \geq 0$ for each $i$ and $\sum_{i=1}^{n} p_i = 1$. Now, at any point in time, the successive slots **40** in the ranked list **42** can be assigned to an alert of mode $i$ by using fair queueing, where the share of each model is given by priority $p_i$. Each model, on average, gets its fair share of the top- $N$ of the ranked list **42.** However, only priority criterion is taken into account at this point.

Now, several more or less independent criteria can be accounted for, including for example: the predictive model **34** that generated the alert (previously called priority), a deadline of an alert **30,** a type of customer contract, a "customer temperature", a number of similar systems that the customer has, modalities for which the current **RME** has expertise, and so forth. As used herein, the term "customer temperature" refers to a measure that expresses how satisfied a customer (i.e., hospital) is with the current maintenance service that it has experienced so far this year. This can for example be measured by the fraction of unplanned downtime. Since the maintenance service contract between the hospital and the maintenance service provider can, amongst others, be defined by an upper bound on the allowed fraction of unplanned downtime, the customer temperature can be measured by the total unplanned downtime that the system has experienced so far during the current year.

In addition, a multi-criteria share is computed for each maintenance alert **30** (represented as alert $a$) given by $(s_{a1}, s_{a2}, s_{a3}, s_{a4}, s_{a5}, s_{a6})$ (e.g., 6 criteria **36**). For each criterion, a relatively small number of values that this criterion can attain are defined. For the predictive models' criterion, there is simply the set of different predictive models **34.** For the deadline criterion, the deadlines can be partitioned into categories as deadline within one week, deadline between one and two weeks, etc. For the customer contract criterion, the number of contract types can be used, and so forth.

For each criterion, a share must be assigned to each of the possible values such that the sum of shares, summed over all values of the given criterion add up to one (1.00). By simply multiplying the shares of all criteria **36** for a given alert **30,** the share that is given to that combination of criterion values is obtained. Using this choice of share, the fair-queueing algorithm can be used again. Each separate criterion the successive values will still receive their fair share in the assignment.

As explained for the single criterion case, one or more values of a criterion do not occur in the current list **42** of alerts. In that case, the shares of the occurring values of this criterion are increased proportionally such that they still add up to one.

For example, a situation can involve two criteria: one with two values $v_{11}$ and $v_{12}$, and one with three values $v_{21}$, $v_{22}$ and $v_{23}$. The shares for the first criterion are given by $s(v_{11}) = 0.6$ and $s(v_{12}) = 0.4$ and the shares for the second criterion are given by $s(v_{21}) = 0.5$, $s(v_{22}) = 0.3$, and $s(v_{23}) = 0.2$. For each of the two criteria, the sum of the shares adds up to one. An alert **30** expressed by a pair $(v_{11}, v_{23})$ is given shares (0.6, 0.2) and the total share for alerts with this pair of values is given by $0.6 \cdot 0.2 = 0.12$. The following matrix (Table 1) shows the total share for alerts for each of the possible value pairs.

Table 1

|  | $v_{11}$ | $v_{12}$ |
|---|---|---|
| $v_{21}$ | 0.30 | 0.20 |
| $v_{22}$ | 0.18 | 0.12 |
| $v_{23}$ | 0.12 | 0.08 |

By summing the columns in Table 1, the total share assigned to $v_{11}$ and $v_{12}$ is still 0.6 and 0.4, respectively. Similarly, summing by the rows in Table 1, the total share assigned to $v_{21}$, $v_{22}$, and $v_{23}$ is still 0.5, 0.3, and 0.2, respectively.

If, for example, each of the six possible value pairs there are several alerts **30,** then successive positions of the ranked list **42** can be determined as given by Table 2:

Table 2

| | | | | | | | |
|---|---|---|---|---|---|---|---|
| 1 | 0.30 - 1.00 | 0.18 | 0.12 | 0.20 | 0.12 | 0.08 | $v_{11}, v_{21}$ |
| 2 | -0.40 | 0.36 | 0.24 | 0.40 - 1.00 | 0.24 | 0.16 | $v_{12}, v_{21}$ |
| 3 | -0.10 | 0.54 - 1.00 | 0.36 | -0.40 | 0.36 | 0.24 | $v_{11}, v_{22}$ |
| 4 | 0.20 | -0.28 | 0.48 - 1.00 | -0.20 | 0.48 | 0.32 | $v_{11}, v_{23}$ |
| 5 | 0.50 | -0.10 | -0.40 | 0.00 | 0.60 - 1.00 | 0.40 | $v_{12}, v_{22}$ |
| 6 | 0.80 - 1.00 | 0.08 | -0.28 | 0.20 | -0.28 | 0.48 | $v_{11}, v_{21}$ |
| 7 | 0.10 | 0.26 | -0.16 | 0.40 | -0.16 | 0.56 - 1.00 | $v_{12}, v_{23}$ |
| 8 | 0.40 | 0.44 | -0.04 | 0.60 - 1.00 | -0.04 | -0.36 | $v_{12}, v_{21}$ |
| 9 | 0.70 - 1.00 | 0.62 | 0.08 | -0.20 | 0.08 | -0.28 | $v_{11}, v_{21}$ |
| 10 | 0.00 | 0.80-1.00 | 0.20 | 0.00 | 0.20 | -0.20 | $v_{11}, v_{22}$ |

Considering each criterion separately, each value of the criterion can obtain its fair share. Except for the last column, the successive columns relate to combinations $(v_{11}, v_{21})$, $(v_{11}, v_{22})$, $(v_{11}, v_{23})$, $(v_{12}, v_{21})$, $(v_{12}, v_{22})$, $(v_{12}, v_{22})$, respectively. The successive entries in these columns show how the credit for such a combination changes for the successive slots. The last column shows the combination to which the successive slots are assigned.

The total number of slots **40** assigned to $v_{11}$ is 6 slots in the first 10 positions and the total number of slots assigned to $v_{12}$ is 4 slots in the first 10 positions. Likewise, the total number of slots assigned to $v_{21}$ is 5 slots, to $v_{22}$ is 3 slots, and to $v_{23}$ is 2 slots.

If, for one or more values of one or more criteria **36,** there are no alerts **30** queued presently, then the shares can be re-weighed for each criterion, such that again the sum of the shares sum up to one. For example, there may be no alerts with $v_{23}$ for the second criterion. In that case, the shares of $v_{21}$ and $v_{22}$ are changed by:

$$s(v_{21}) \leftarrow \frac{s(v_{21})}{s(v_{21}) + s(v_{22})} \quad s(v_{22}) \leftarrow \frac{s(v_{22})}{s(v_{21}) + s(v_{22})}$$

Hence, $s(v_{21}) = \frac{0.5}{0.5+0.3} = 5/8$ and $s(v_{22}) = \frac{0.3}{0.5+0.3} = 3/8$, and Table 1 is updated into Table 3.

Table 3

| | $v_{11}$ | $v_{12}$ |
|---|---|---|
| $v_{21}$ | 0.375 | 0.250 |
| $v_{22}$ | 0.225 | 0.150 |
| $v_{23}$ | 0.000 | 0.000 |

The multi-criterion weighted fair queueing process **38** can also be used to adapt the ranked list **42** of alerts to the specific **RME** that is currently handling the list. As such, modality can be added as an additional criterion. If the **RME** has expertise in modalities CT and interventional X-ray (IXR), but not in MR, share values can be assigned as $s(CT) = 0.6$, $s(IXR) = 0.4$, and $s(MR) = 0.0$, while for another **RME** with most expertise in MR and less expertise in CT, share values can be assign as $S(CT) = 0.25$, $S(IXR) = 0.0$, and $s(MR) = 0.75$.

**Claims**

1. A non-transitory computer readable medium (**18**) storing instructions which, when executed by at least one electronic processor (**16**), cause the at least one electronic processor (**16**) to perform a method (**100**) of generating a ranked list (**42**) of alerts (**30**) from files (**32**) of electronic devices (**12**), the method (**100**) comprising:

   applying predictive models (**34**) to data of the files to generate maintenance alerts for the electronic devices;
   assigning discrete values to a set of criteria (**36**) for each maintenance alert, and computing for each maintenance alert a share corresponding to a priority of the maintenance alert, whereby the share for each maintenance alert is computed as a product of the assigned discrete values;
   performing a credit-based queuing process (**38**) to assign the maintenance alerts to slots of a ranked list, wherein in the credit-based queuing process the maintenance alerts accumulate credits in accord with the shares computed for the respective maintenance alerts; and
   displaying, on a display device (**24**) operatively connected with the at least one electronic processor, the maintenance alerts as the ranked list in accordance with the assignments of the maintenance alerts to the slots of the ranked list.

2. The non-transitory computer readable medium (**18**) of claim 1, wherein each criterion of the set of criteria (**36**) assume discrete values of a set of discrete values in which the sum of the discrete values of the set of discrete values equals one.

3. The non-transitory computer readable medium (**18**) of claim 1, wherein the method (**100**) further comprises:
   recomputing the set of discrete values for a single criterion of the set of the criteria (**36**) while ensuring the sum of the discrete values of the set of discrete values remains equal to one.

4. The non-transitory computer readable medium (**18**) of any one of claims 1-3, wherein the credit-based queuing process (**38**) is a credit-based fair queuing process in which each iteration of the credit-based fair queuing process includes assigning the maintenance alerts to the slots of the ranked list by:

   increasing the credit for each maintenance alert (**30**) by an amount equal to the share computed for the maintenance alert;
   assigning the maintenance alert having a highest accumulated credit to a current slot of the ranked list; and
   after the assigning, decreasing the credit of the maintenance alert assigned to the current slot by a predetermined amount.

5. The non-transitory computer readable medium (**18**) of claim 4, wherein the predetermined amount comprises 1.00.

6. The non-transitory computer readable medium (**18**) of any one of claims 1-5, wherein the set of criteria (**36**) include (1) a predictive model criterion whose assigned value for a maintenance alert (**30**) corresponds to the predictive model that generated the maintenance alert and (2) a timeframe assigned to the maintenance alert by the predictive model.

7. The non-transitory computer readable medium (**18**) of claim 6, wherein the set of criteria (**36**) further include one or more of:

   a criterion based on a maintenance contract for an electronic device (**2**) from the plurality of electronic devices (12) for which a maintenance alert is generated;
   a criterion based on a satisfaction level of an owner of the electronic device for which the maintenance alert is generated.

8. The non-transitory computer readable medium (**18**) of claim 7, wherein the criterion based on a satisfaction level of an owner of an electronic device (**2**) from the plurality of electronic devices (12) for which a maintenance alert (**30**) is generated includes a potential downtime during servicing of the electronic device, and the credit-based queuing process includes:
   analyzing the generated maintenance alert with machine learning algorithms to predict possible downtime of the electronic device.

9. The non-transitory computer readable medium (**18**) of any one of claims 6-8, wherein the electronic devices (**12**) comprise medical imaging devices of a plurality of different imaging modalities, and the set of criteria (**36**) further includes:

a criterion based on the imaging modality of the medical imaging device for which the maintenance alert is generated.

10. The non-transitory computer readable medium (**18**) of any one of claims 6-9, wherein the electronic devices (**12**) comprise a plurality of medical imaging devices (**2**), and the set of criteria (**36**) further includes a volume of examinations using the plurality of medical imaging device relative to the number of medical imaging devices.

11. The non-transitory computer readable medium (**18**) of any one of claims 1-10, wherein the method (**100**) further includes:

discarding maintenance alerts (**30**) having a share of zero.

12. The non-transitory computer readable medium (**18**) of any one of claims 1-11, wherein the method (**100**) further includes:

removing maintenance alerts (**30**) having a share of indeterminable value.

13. A method (**100**) of generating a ranked list (**42**) of alerts (**30**) from files (**32**) of electronic devices (**12**), the method comprising:

applying predictive models (**34**) to data of the files to generate maintenance alerts for the electronic devices;
assigning discrete values to a set of criteria (**36**) for each maintenance alert, and computing for each maintenance alert a share corresponding to a priority of the maintenance alert, whereby the share for each maintenance alert is computed as a product of the assigned discrete values, wherein the set of criteria include (1) a predictive model criterion whose assigned value for a maintenance alert corresponds to the predictive model that generated the maintenance alert and (2) a timeframe assigned to the maintenance alert by the predictive model;
performing a credit-based queuing process (**38**) to assign the maintenance alerts to slots of a ranked list, wherein in the credit-based queuing process the maintenance alerts accumulate credits in accord with the shares computed for the respective maintenance alerts, wherein the credit-based queuing process (**38**) is a credit-based fair queuing process in which each iteration of the credit-based fair queuing process includes:

increasing the credit for each maintenance alert (**30**) by an amount equal to the share computed for the maintenance alert;
assigning the maintenance alert having a highest accumulated credit to a current slot of the ranked list; and
after the assigning, decreasing the credit of the maintenance alert assigned to the current slot by a predetermined amount; and

displaying, on a display device (**24**) operatively connected with the at least one electronic processor, the maintenance alerts as the ranked list in accordance with the assignments of the maintenance alerts to the slots of the ranked list.

**Patentansprüche**

1. Nichtflüchtiges computerlesbares Medium (**18**), das Anweisungen speichert, die, wenn sie von mindestens einem elektronischen Prozessor (**16**) ausgeführt werden, den mindestens einen elektronischen Prozessor (**16**) veranlassen, ein Verfahren (**100**) zum Erzeugen einer Rangliste (**42**) von Alarmen (**30**) aus Dateien (**32**) von elektronischen Vorrichtungen (**12**) durchzuführen, wobei das Verfahren (**100**) umfasst:

Anwenden von Vorhersagemodellen (**34**) auf Daten der Dateien, um Wartungsalarme für die elektronischen Vorrichtungen zu erzeugen;
Zuweisen von diskreten Werten zu einer Reihe von Kriterien (**36**) für jeden Wartungsalarm und Berechnen für jeden Wartungsalarm eines Anteils, der einer Priorität des Wartungsalarms entspricht, wobei der Anteil für jeden Wartungsalarm als Produkt der zugewiesenen diskreten Werte berechnet wird;
Durchführen eines kreditbasierten Warteschlangenprozesses (**38**), um die Wartungsalarme zu Slots einer Rangliste zuzuweisen, wobei in dem kreditbasierten Warteschlangenprozess die Wartungsalarme Kredite in Übereinstimmung mit den Anteilen, die für die jeweiligen Wartungsalarme berechnet wurden, ansammeln; und
Anzeigen der Wartungsalarme als Rangliste in Übereinstimmung mit den Zuweisungen der Wartungsalarme zu den Slots der Rangliste auf einer Anzeigevorrichtung (**24**), die mit dem mindestens einen elektronischen Prozessor wirkverbunden ist.

2. Nichtflüchtiges computerlesbares Medium (**18**) nach Anspruch 1, wobei jedes Kriterium der Reihe von Kriterien (**36**) diskrete Werte einer Reihe von diskreten Werten annimmt, bei denen die Summe der diskreten Werte der Reihe von diskreten Werten gleich eins ist.

3. Nichtflüchtiges computerlesbares Medium (**18**) nach Anspruch 1, wobei das Verfahren (**100**) weiter umfasst: Neuberechnen der Reihe von diskreten Werten für ein einzelnes Kriterium der Reihe der Kriterien (**36**), während sichergestellt wird, dass die Summe der diskreten Werte der Reihe von diskreten Werten gleich eins bleibt.

4. Nichtflüchtiges computerlesbares Medium (**18**) nach einem der Ansprüche 1-3, wobei der kreditbasierte Warteschlangenprozess (**38**) ein kreditbasierter fairer Warteschlangenprozess ist, bei dem jede Iteration des kreditbasierten fairen Warteschlangenprozesses das Zuweisen der Wartungsalarme zu den Slots der Rangliste beinhaltet durch:

Erhöhen des Kredits für jeden Wartungsalarm (**30**) um einen Betrag, der gleich dem für den Wartungsalarm berechneten Anteil ist;
Zuweisen des Wartungsalarms, der einen höchsten angesammelten Kredit aufweist, zu einem aktuellen Slot der Rangliste; und
nach dem Zuweisen, Verringern des Kredits des dem aktuellen Slot zugewiesenen Wartungsalarms um einen vorbestimmten Betrag.

5. Nichtflüchtiges computerlesbares Medium (**18**) nach Anspruch 4, wobei der vorbestimmte Betrag 1,00 umfasst.

6. Nichtflüchtiges computerlesbares Medium (**18**) nach einem der Ansprüche 1-5, wobei die Reihe von Kriterien (**36**) (1) ein Vorhersagemodellkriterium beinhaltet, dessen zugewiesener Wert für einen Wartungsalarm (**30**) dem Vorhersagemodell entspricht, das den Wartungsalarm erzeugt hat, und (2) einen Zeitrahmen, der dem Wartungsalarm durch das Vorhersagemodell zugewiesen wurde.

7. Nichtflüchtiges computerlesbares Medium (**18**) nach Anspruch 6, wobei die Reihe von Kriterien (**36**) weiter eines oder mehr beinhaltet von:

einem Kriterium basierend auf einem Wartungsvertrag für eine elektronische Vorrichtung (**2**) aus der Vielzahl von elektronischen Vorrichtungen (12), für die ein Wartungsalarm erzeugt wird;
einem Kriterium basierend auf einem Zufriedenheitsgrad eines Besitzers der elektronischen Vorrichtung, für die der Wartungsalarm erzeugt wird.

8. Nichtflüchtiges computerlesbares Medium (**18**) nach Anspruch 7, wobei das Kriterium basierend auf einem Zufriedenheitsgrad eines Besitzers einer elektronischen Vorrichtung (**2**) aus der Vielzahl von elektronischen Vorrichtungen (12), für die ein Wartungsalarm (**30**) erzeugt wird, eine potenzielle Ausfallzeit während der Instandhaltung der elektronischen Vorrichtung beinhaltet, und der kreditbasierte Warteschlangenprozess beinhaltet: Analysieren des erzeugten Wartungsalarms mit maschinellen Lernalgorithmen, um mögliche Ausfallzeiten der elektronischen Vorrichtung vorherzusagen.

9. Nichtflüchtiges computerlesbares Medium (**18**) nach einem der Ansprüche 6-8, wobei die elektronischen Vorrichtungen (**12**) medizinische Bildgebungsvorrichtungen einer Vielzahl unterschiedlicher Bildgebungsmodalitäten umfassen und die Reihe von Kriterien (**36**) weiter beinhaltet:
ein Kriterium basierend auf der Bildgebungsmodalität der medizinischen Bildgebungsvorrichtung, für die der Wartungsalarm erzeugt wird.

10. Nichtflüchtiges computerlesbares Medium (**18**) nach einem der Ansprüche 6-9, wobei die elektronischen Vorrichtungen (**12**) eine Vielzahl von medizinischen Bildgebungsvorrichtungen (**2**) umfassen und die Reihe von Kriterien (**36**) weiter ein Untersuchungsvolumen unter Verwendung der Vielzahl von medizinischen Bildgebungsvorrichtungen in Bezug zur Anzahl medizinischer Bildgebungsvorrichtungen beinhaltet.

11. Nichtflüchtiges computerlesbares Medium (**18**) nach einem der Ansprüche 1-10, wobei das Verfahren (**100**) weiter beinhaltet:
Verwerfen von Wartungsalarmen (**30**), die einen Anteil von Null aufweisen.

**12.** Nichtflüchtiges computerlesbares Medium (**18**) nach einem der Ansprüche 1-11, wobei das Verfahren (**100**) weiter beinhaltet:
Entfernen von Wartungsalarmen (**30**), die einen Anteil mit unbestimmbarem Wert aufweisen.

**13.** Verfahren (**100**) zum Erzeugen einer Rangliste (**42**) von Alarmen (**30**) aus Dateien (**32**) von elektronischen Vorrichtungen (**12**), wobei das Verfahren umfasst:

Anwenden von Vorhersagemodellen (**34**) auf Daten der Dateien, um Wartungsalarme für die elektronischen Vorrichtungen zu erzeugen;
Zuweisen von diskreten Werten zu einer Reihe von Kriterien (**36**) für jeden Wartungsalarm, und Berechnen für jeden Wartungsalarm eines Anteils, der einer Priorität des Wartungsalarms entspricht, wobei der Anteil für jeden Wartungsalarm als Produkt der zugewiesenen diskreten Werte berechnet wird, wobei die Reihe von Kriterien (1) ein Vorhersagemodellkriterium beinhalten, dessen zugewiesener Wert für einen Wartungsalarm dem Vorhersagemodell entspricht, das den Wartungsalarm erzeugt hat, und (2) einen Zeitrahmen, der dem Wartungsalarm durch das Vorhersagemodell zugewiesen wurde;
Durchführen eines kreditbasierten Warteschlangenprozesses (**38**), um die Wartungsalarme zu Slots einer Rangliste zuzuweisen, wobei in dem kreditbasierten Warteschlangenprozess die Wartungsalarme Kredite in Übereinstimmung mit den Anteilen, die für die jeweiligen Wartungsalarme berechnet wurden, ansammeln; wobei der kreditbasierte Warteschlangenprozess (**38**) ein kreditbasierter fairer Warteschlangenprozess ist, bei dem jede Iteration des kreditbasierten fairen Warteschlangenprozesses beinhaltet:

Erhöhen des Kredits für jeden Wartungsalarm (**30**) um einen Betrag, der gleich dem für den Wartungsalarm berechneten Anteil ist;
Zuweisen des Wartungsalarms, der einen höchsten angesammelten Kredit aufweist, zu einem aktuellen Slot der Rangliste; und
nach dem Zuweisen, Verringern des Kredits des dem aktuellen Slot zugewiesenen Wartungsalarms um einen vorbestimmten Betrag; und

Anzeigen der Wartungsalarme als Rangliste in Übereinstimmung mit den Zuweisungen der Wartungsalarme zu den Slots der Rangliste auf einer Anzeigevorrichtung (**24**), die mit dem mindestens einen elektronischen Prozessor wirkverbunden ist.

**Revendications**

**1.** Support non transitoire lisible par ordinateur (**18**) stockant des instructions qui, lorsqu'elles sont exécutées par au moins un processeur électronique (**16**), amènent le au moins un processeur électronique (**16**) à exécuter un procédé (**100**) de génération d'une liste classée (**42**) d'alertes (**30**) à partir de fichiers (**32**) de dispositifs électroniques (**12**), le procédé (**100**) comprenant :

l'application de modèles prédictifs (**34**) aux données des fichiers pour générer des alertes de maintenance pour les dispositifs électroniques ;
l'attribution de valeurs discrètes à un ensemble de critères (**36**) pour chaque alerte de maintenance, et le calcul pour chaque alerte de maintenance d'une part correspondant à une priorité de l'alerte de maintenance, selon lequel la part pour chaque alerte de maintenance est calculée comme un produit des valeurs discrètes attribuées ;
l'exécution d'un processus de mise en file d'attente basé sur le crédit (**38**) pour attribuer les alertes de maintenance à des emplacements d'une liste classée, dans lequel, dans le processus de mise en file d'attente basé sur le crédit, les alertes de maintenance accumulent des crédits en fonction des parts calculées pour les alertes de maintenance respectives ; et
l'affichage, sur un dispositif d'affichage (**24**) connecté opérationnellement au au moins un processeur électronique, des alertes de maintenance sous forme de liste classée en fonction des affectations des alertes de maintenance aux emplacements de la liste classée.

**2.** Support non transitoire lisible par ordinateur (**18**) selon la revendication 1, dans lequel chaque critère de l'ensemble de critères (**36**) prend des valeurs discrètes d'un ensemble de valeurs discrètes dans lesquelles la somme des valeurs discrètes de l'ensemble de valeurs discrètes est égale à un.

**3.** Support non transitoire lisible par ordinateur (**18**) selon la revendication 1, dans lequel le procédé (**100**) comprend en outre :
le recalcul de l'ensemble des valeurs discrètes pour un seul critère de l'ensemble des critères (**36**) tout en s'assurant que la somme des valeurs discrètes de l'ensemble des valeurs discrètes reste égale à un.

**4.** Support non transitoire lisible par ordinateur (**18**) selon l'une quelconque des revendications 1-3, dans lequel le processus de mise en file d'attente basé sur le crédit (**38**) est un processus de mise en file d'attente équitable basé sur le crédit dans lequel chaque itération du processus de mise en file d'attente équitable basé sur le crédit inclut l'attribution des alertes de maintenance aux emplacements de la liste classée par :

l'augmentation du crédit pour chaque alerte de maintenance (**30**) d'une quantité égale à la part calculée pour l'alerte de maintenance ;
l'attribution de l'alerte de maintenance présentant le crédit accumulé le plus élevé à un emplacement actuel de la liste classée ; et
après l'attribution, la diminution du crédit de l'alerte de maintenance attribuée à l'emplacement actuel d'une quantité prédéterminée.

**5.** Support non transitoire lisible par ordinateur (**18**) selon la revendication 4, dans lequel la quantité prédéterminée comprend 1,00.

**6.** Support non transitoire lisible par ordinateur (**18**) selon l'une quelconque des revendications 1-5, dans lequel l'ensemble de critères (**36**) inclut (1) un critère de modèle prédictif dont la valeur attribuée pour une alerte de maintenance (**30**) correspond au modèle prédictif qui a généré l'alerte de maintenance et (2) un délai attribué à l'alerte de maintenance par le modèle prédictif.

**7.** Support non transitoire lisible par ordinateur (**18**) selon la revendication 6, dans lequel l'ensemble de critères (**36**) inclut en outre un ou plusieurs parmi :

un critère basé sur un contrat de maintenance pour un dispositif électronique (**2**) parmi la pluralité de dispositifs électroniques (12) pour lesquels une alerte de maintenance est générée ;
un critère basé sur le niveau de satisfaction d'un propriétaire du dispositif électronique pour lequel l'alerte de maintenance est générée.

**8.** Support non transitoire lisible par ordinateur (**18**) selon la revendication 7, dans lequel le critère basé sur un niveau de satisfaction d'un propriétaire d'un dispositif électronique (**2**) parmi la pluralité de dispositifs électroniques (12) pour lesquels une alerte de maintenance (**30**) est générée inclut un temps d'arrêt potentiel pendant l'entretien du dispositif électronique, et le processus de mise en file d'attente basé sur le crédit inclut :
l'analyse de l'alerte de maintenance générée avec des algorithmes d'apprentissage automatique pour prédire les éventuels temps d'arrêt du dispositif électronique.

**9.** Support non transitoire lisible par ordinateur (**18**) selon l'une quelconque des revendications 6-8, dans lequel les dispositifs électroniques (12) comprennent des dispositifs d'imagerie médicale d'une pluralité de modalités d'imagerie différentes, et l'ensemble de critères (**36**) inclut en outre :
un critère basé sur la modalité d'imagerie du dispositif d'imagerie médicale pour lequel l'alerte de maintenance est générée.

**10.** Support non transitoire lisible par ordinateur (**18**) selon l'une quelconque des revendications 6-9, dans lequel les dispositifs électroniques (12) comprennent une pluralité de dispositifs d'imagerie médicale (**2**), et l'ensemble de critères (**36**) inclut en outre un volume d'examens utilisant la pluralité de dispositifs d'imagerie médicale par rapport au nombre de dispositifs d'imagerie médicale.

**11.** Support non transitoire lisible par ordinateur (**18**) selon l'une quelconque des revendications 1-10, dans lequel le procédé (**100**) inclut en outre :
le rejet des alertes de maintenance (**30**) présentant une part de zéro.

**12.** Support non transitoire lisible par ordinateur (**18**) selon l'une quelconque des revendications 1-11, dans lequel le procédé (**100**) inclut en outre :
la suppression des alertes de maintenance (**30**) présentant une part de valeur indéterminable.

13. Procédé (**100**) de génération d'une liste classée (**42**) d'alertes (**30**) à partir de fichiers (**32**) de dispositifs électroniques (**12**), le procédé comprenant :

l'application de modèles prédictifs (**34**) aux données des fichiers pour générer des alertes de maintenance pour les dispositifs électroniques ;

l'attribution de valeurs discrètes à un ensemble de critères (**36**) pour chaque alerte de maintenance, et le calcul pour chaque alerte de maintenance d'une part correspondant à une priorité de l'alerte de maintenance, selon lequel la part pour chaque alerte de maintenance est calculée comme un produit des valeurs discrètes attribuées, dans lequel l'ensemble de critères inclut (1) un critère de modèle prédictif dont la valeur attribuée pour une alerte de maintenance correspond au modèle prédictif qui a généré l'alerte de maintenance et (2) un délai attribué à l'alerte de maintenance par le modèle prédictif ;

l'exécution d'un processus de mise en file d'attente basé sur le crédit (**38**) pour attribuer les alertes de maintenance à des emplacements d'une liste classée, dans lequel, dans le processus de mise en file d'attente basé sur le crédit, les alertes de maintenance accumulent des crédits en fonction des parts calculées pour les alertes de maintenance respectives ; dans lequel le processus de file d'attente basé sur le crédit (**38**) est un processus de file d'attente équitable basé sur le crédit dans lequel chaque itération du processus de file d'attente équitable basé sur le crédit inclut :

l'augmentation du crédit pour chaque alerte de maintenance (**30**) d'une quantité égale à la part calculée pour l'alerte de maintenance ;

l'attribution de l'alerte de maintenance présentant le crédit accumulé le plus élevé à un emplacement actuel de la liste classée ; et

après l'attribution, la diminution du crédit de l'alerte de maintenance attribuée à l'emplacement actuel d'une quantité prédéterminée ; et

l'affichage, sur un dispositif d'affichage (**24**) connecté opérationnellement au au moins un processeur électronique, des alertes de maintenance sous forme de liste classée en fonction des affectations des alertes de maintenance aux emplacements de la liste classée.

**Figure 1**

**Figure 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3379356 A1